# EUROPEAN PATENT APPLICATION

(11) **EP 3 943 068 A1**
(43) Date of publication of application: **26.01.2022**
(21) Application number: 20305842.5
(22) Date of filing: 22.07.2020
(51) Int. Cl.: A61K 9/08, A61K 47/12, A61K 31/225, A61M 5/31

(54) **SUXAMETHONIUM COMPOSITION AND PREFILLED SYRINGE THEREOF**

(71) Applicant: Laboratoire Aguettant, 69007 Lyon (FR)
(72) Inventor: TONNAR, Jeff, 69007 Lyon (FR); BERGER LACOUR, Sandrine, 69160 Tassin-la-Demi-Lune (FR)
(74) Representative: Icosa

(57) **Abstract**

The present invention relates to a suxamethonium pharmaceutical composition comprising a succinic acid buffer. It also relates to a prefilled syringe thereof. The composition and the prefilled syringe of the invention are useful as ready-to-use formulations, especially in emergency conditions, and present long shelf lives, even at room temperature.

## Description

### FIELD OF INVENTION

The present invention relates to a ready-to-use suxamethonium pharmaceutical composition and prefilled syringe thereof, especially useful in emergency conditions.

### BACKGROUND OF INVENTION

Suxamethonium, also called succinylcholine, is a short-acting depolarizing neuromuscular blocking agent. Suxamethonium is clinically used to facilitate endotracheal intubation and mechanical ventilation during general anesthesia or in emergency conditions. Suxamethonium is administered to patients by parenteral route, preferably by intravenous injection.

To date, the available commercial suxamethonium compositions are aqueous solutions with a concentration in suxamethonium chloride of 20 mg/ml or 50 mg/ml. One of the most widespread product on the market is a 100 mg/2 ml presentation. However, in practice, professionals often use diluted solutions to achieve a concentration of 10 mg/ml. Therefore, they need to dilute the commercial solutions, which is associated with risks of medical errors, contamination concerns and shelf life of such diluted solutions.

Consequently, there is a need for ready-to-use suxamethonium compositions at more diluted concentrations in suxamethonium, namely around 10 mg/ml.

It is often observed that dilution of given compositions may negatively impact the stability of their components. Besides, suxamethonium is known to be a quite unstable molecule. The degradation of suxamethonium proceeds through a hydrolysis of the ester bonds. The hydrolysis yields succinylmonocholine, choline and succinic acid. The initial pH of the composition has thus to be carefully controlled in order to ensure chemical stability overtime. For example, the Applicant evidenced that the degradation is faster at pH 5.0 than at pH 3.5. Moreover, in suxamethonium aqueous solutions, the formation of succinic acid upon degradation of suxamethonium leads to pH decrease, which in turn accelerates the kinetic of degradation of suxamethonium.

Consequently, a simple dilution of existing concentrated aqueous solutions of suxamethonium was not found to be sufficient to ensure the stability of a 10 mg/ml suxamethonium composition overtime to provide a sufficient shelf life, both at 2-8°C and at room temperature. Therefore, the formulation of the 10 mg/ml suxamethonium composition needed to be optimized.

The Applicant evidenced that buffering a 10 mg/ml suxamethonium composition at a pH ranging from 3.0 to 4.5 with succinic acid at a concentration ranging from 5 mM to 20 mM enables to ensure its stability overtime both at 2-8°C and at room temperature. The selection of this range of concentration of succinic acid was essential since it was evidenced that the composition should comprise enough but not too much succinic acid in order to achieve expected stability during the shelf life. Higher succinic acid content increases the degradation kinetic of suxamethonium. Lower succinic acid content negatively impacts the buffering of the composition and the degradation of suxamethonium.

Currently commercialized solutions for injection of suxamethonium are manufactured by aseptic process and final sterile filtration. Nevertheless, according to International Council for Harmonisation (ICH) and European Medicine Agency (EMA) guidelines, final heat sterilization should however be privileged in order to ensure a high security for patients and insurance of sterility of the final drug product.

However, suxamethonium is known to be sensitive to temperature conditions and to degrade when exposed to heat. Schmutz et al. studied the stability of a suxamethonium chloride compositions, especially towards steam sterilization (Schmutz et al., Am. J. Hosp. Pharm., 1991, 48(3), 501-506). The aqueous compositions comprised the equivalent of 10 mg/ml of suxamethonium chloride anhydrous, sodium chloride as tonicity agent, methyl-4-hydroxybenzoate as preservative agent, having a pH of 4.2 or adjusted to 3.0 with hydrochloric acid, without any buffer. Schmutz et al. state that standard method of steam sterilization at 121°C in autoclave is not adapted to tested suxamethonium compositions since it increases their degradation. Instead, they recommend a steam sterilization at only 100°C for 30 minutes to limit suxamethonium degradation. However, such conditions of sterilization are close to no efficacy. Indeed, it is estimated that 1 minute at 121°C corresponds to the same F0 value than 126 minutes at 100°C. Consequently, the sterilization at 100°C for 30 minutes proposed by Schmutz et al. corresponds to a F0 of 0.25, while specifications for such sterile injectable products recommend a F0 of more than 15 in order to guarantee a good sterility assurance. This can explain why the tested composition comprises an antimicrobial preservative agent (methyl-4-hydroxybenzoate).

Despite this negative incentive relative to heat sterilization of suxamethonium compositions, and in order to ensure maximum patient safety, the Applicant herein provides a process of steam sterilization at 121°C of the suxamethonium composition of the invention. In order to compensate the degradation of suxamethonium during sterilization, an overage of suxamethonium is used during the manufacturing of the composition. The degradation products formed during sterilization are exactly the same as those formed during degradation during normal storage and correspond to the natural metabolites of suxamethonium (upon degradation in vivo). A safety assessment has proved (based on bibliographic data) that the degradation products at the maximum concentrations achievable during sterilization do not present a significant pharmacological or toxicological effect. They are safe and without danger to be administered at these concentrations.

In order to provide ready-to use 10 mg/ml suxamethonium compositions, the Applicant also developed a prefilled syringe comprising the composition of the invention, to allow to the emergency professionals to save time to treat their patients and to decrease the number of handlings and consequently the risk of microbial contamination.

Prefilled glass syringes containing suxamethonium compositions were described in WO2019/177725. Exemplified composition comprises 20 mg/ml suxamethonium chloride, sodium chloride as tonicity agent, with a pH adjusted to about 3.6 with HCl or NaOH, without any buffer. Nevertheless, as mentioned in WO2019/177725, glass syringes present the drawback of being breakable.

In the present application, the prefilled syringe is advantageously a plastic syringe, preferably in polypropylene. The use of polypropylene reduces particulate contamination (compared to glass containers) and is non-breakable.

### SUMMARY

This invention thus relates to an aqueous pharmaceutical composition for parenteral administration, comprising:
- suxamethonium chloride at a concentration ranging from 8 mg/ml to 12 mg/ml; and
- succinic acid at a concentration ranging from 5 mM to 20 mM;
and having a pH ranging from 3.0 to 4.5.

In one embodiment, the concentration of suxamethonium chloride in the composition corresponds to 10 mg/ml of suxamethonium chloride anhydrous. In one embodiment, succinic acid is present at a concentration of 6 mM. In one embodiment, the pH is ranging from 3.4 to 3.8. In one embodiment, the composition has an osmolality ranging from 250 mOsm/kg to 350 mOsm/kg, preferably an osmolality of about 300 mOsm/kg. In one embodiment, the water used to form the aqueous composition is water for injection grade.

In one embodiment, the composition further comprises sodium chloride, preferably at a concentration of about 7 mg/ml. In one embodiment, the composition further comprises a pH adjusting agent, preferably selected from hydrochloric acid, sodium hydroxide and mixtures thereof.

In one embodiment, the composition is sterile, the sterility being obtained preferably by heat sterilization.

The invention also provides a suxamethonium prefilled syringe comprising an aqueous pharmaceutical composition according to the invention.

In one embodiment, the syringe is a plastic syringe; preferably a syringe made of polypropylene, cyclic olefin copolymer and/or cyclic olefin polymer. In one embodiment, the syringe has a total volume of selected from 5 ml, 10 ml, 20 ml and 50 ml.

In one embodiment, the suxamethonium prefilled syringe is sterilized by terminal heat sterilization.

The invention further relates to a process for sterilizing a suxamethonium prefilled syringe, comprising:
- providing a suxamethonium prefilled syringe according to the invention;
- subjecting the suxamethonium prefilled syringe to steam sterilization at a temperature ranging from 118°C to 125°C, with F0 ranging from 8 to 30.

### DEFINITIONS

In the present invention, the following terms have the following meanings:
- "**Suxamethonium**" refers to succinylcholine, of chemical name 2,2'-[(1,4-dioxobutane-1,4-diyl)bis(oxy)]bis(N,N,N-trimethylethanaminium. Suxamethonium can be under the form of a salt, preferably a pharmaceutically acceptable salt, such as chloride, bromine or iodide, preferably chloride.
- "**Pharmaceutical composition**" refers to a composition whose components are compatible with each other and not deleterious to the subject to which it is administered.
- "**Parenteral administration**" refers to drug administration by injection, infusion, and implantation or by some other route other than the alimentary canal. Parenteral administration includes intravenous (IV), intramuscular (IM), subcutaneous (SC) and intradermal (ID) administrations.
- "**pH adjusting agent**" refers to a substance that enable to adjust the pH of a composition. The pH adjusting agent can be an acidifying or alkalizing agent. Acidifying agents, such as hydrochloric acid, are used to lower the pH and alkalizing agents, such as sodium hydroxide, are used to increase the pH.
- "**Water for injection grade**" refers to water of very high quality without significant contamination, as defined by the European or US pharmacopeia. Water for injection is generally obtained by distillation or reverse osmosis.
- **"Osmolality"** of a solution refers to the concentration of osmotically active molecules in that solution and is expressed as the number of molecules of solute per kilogram of solvent.
- **"Prefilled syringe"** refers to a ready-to-use non-reusable product. This type of syringe is filled with the desired liquid and sterilized industrially by the pharmaceutical laboratory. Sterility of the container/contents assembly is achieved either by filling under aseptic conditions syringes whose components have been pre-sterilized, or by steam-sterilizing the container/contents assembly at the end.
- **"Sterile composition"** refers to a composition, which is free of bacteria or other microorganisms, and that meets usual Pharmacopeia requirements for sterility.
- **"Sterilization"** refers to any physical or chemical process which destroys all life forms, with special regard to microorganisms (including bacteria and sporogenous forms), and inactivates viruses.
- **"Terminal sterilization"** refers to a sterilization process that takes place after that the product to be sterilized has been filled into at least its primary packaging. Terminal sterilization presents the advantage of avoiding further opportunities for contamination of the product due to human intervention.
- **"Heat sterilization"** refers to a sterilization process achieved by exposing the product to be sterilized to heat; **"steam sterilization"** refers to a sterilization process achieved by exposing the product to be sterilized with saturated steam.
- **"Ready-to-use composition"** refers to a composition that is suitable for administration to a patient without dilution, and preferably without any other handling. Preferably, a ready-to-use composition is present in a prefilled syringe and there is no need to remove the composition from a storage container in order to be able to administer the composition to a patient.
- **"About"** preceding a figure means plus or less 10% of the value of said figure.

### DETAILED DESCRIPTION

### Suxamethonium composition

This invention thus relates to a suxamethonium composition, especially a pharmaceutical composition comprising suxamethonium.

In one embodiment, the suxamethonium composition of the invention is an aqueous composition, i.e. a composition comprising suxamethonium in an aqueous solvent, preferably water.

In one embodiment, the suxamethonium composition of the invention is suitable for parenteral administration. In one embodiment, a composition which is suitable for parenteral administration complies with the requirement associated with this delivery route defined by the US Food and Drug Administration (FDA) and/or the European Medicines Agency (EMA).

In one embodiment, suxamethonium is present in the composition of the invention as suxamethonium chloride salt, which is the salt of suxamethonium described in the European and USP pharmacopeia monographs.

In one embodiment, the suxamethonium composition of the invention comprises suxamethonium and succinic acid. In one embodiment, the aqueous pharmaceutical composition of the invention comprises suxamethonium chloride and succinic acid.

In one embodiment, the suxamethonium composition of the invention has a pH ranging preferably from 3.0 to 4.5. In one embodiment, the suxamethonium composition of the invention is an aqueous composition comprising suxamethonium chloride and succinic acid and has a pH ranging from 3.0 to 4.5.

In one embodiment, the invention provides an aqueous pharmaceutical composition for parenteral administration, comprising:
- suxamethonium chloride at a concentration ranging from 8 mg/ml to 12 mg/ml; and
- succinic acid at a concentration ranging from 5 mM to 20 mM;
and having a pH ranging from 3.0 to 4.5.

In one embodiment, the aqueous composition of the invention can be manufactured using suxamethonium chloride anhydrous or suxamethonium chloride dihydrate. Suxamethonium chloride dihydrate API is described in the European Pharmacopoeia and suxamethonium chloride anhydrous API is described in the USP pharmacopeia. The hydration number of the suxamethonium chloride used to manufacture the composition of the invention does not impact the resulting aqueous composition since once in solution, hydration is identical, no matter the initial hydration number.

In one embodiment, the aqueous composition of the invention comprises suxamethonium chloride at a concentration ranging from 8 mg/ml to 12 mg/ml; preferably from 9 mg/ml to 11 mg/ml; more preferably about 10 mg/ml. In one embodiment, the aqueous composition of the invention comprises suxamethonium chloride at a concentration of 8.0 mg/ml, 8.5 mg/ml, 9.0 mg/ml, 9.5 mg/ml, 10.0 mg/ml, 10.5 mg/ml, 11.0 mg/ml, 11.5 mg/ml or 12.0 mg/ml. In one embodiment, the composition of the invention comprises suxamethonium chloride anhydrous, present at a concentration of about 10 mg/ml, corresponding to about 11 mg/ml of suxamethonium chloride dihydrate. In another embodiment, the composition of the invention comprises suxamethonium chloride dihydrate, present at a concentration of about 10 mg/ml, corresponding to about 9.1 mg/ml of suxamethonium chloride anhydrous.

With such concentrations in suxamethonium chloride, the composition of the invention is ready to be used directly for administration to the patient, without the need to reconstitute or dilute the composition prior to administration.

In one embodiment, the aqueous composition of the invention comprises succinic acid. Succinic acid is an approved and widely accepted pharmaceutical excipient, used in the composition of the invention as a buffering agent. Succinic acid is also one of the degradation products and metabolites of suxamethonium chloride. Advantageously, the presence of succinic acid in the concentration ranges used in the composition of the invention avoids pH modification during the shelf life of the suxamethonium composition and ensures stability. As evidenced in the experimental part below, the composition should comprise enough but not too much succinic acid in order to achieve expected stability. In one embodiment, the composition comprises succinic acid at a concentration ranging from 5 mM to 20 mM; preferably from 5 mM to 15 mM; from 5 mM to 10 mM; from 5 mM to 7 mM; more preferably about 6 mM. In one embodiment, the composition comprises succinic acid at a concentration of 5 mM, 6 mM, 7 mM, 8 mM, 9 mM, 10 mM, 11 mM, 12 mM, 13 mM, 14 mM, 15 mM, 16 mM, 17 mM, 18 mM, 19 mM or 20 mM.

In one embodiment, the aqueous composition of the invention has a pH ranging from 3.0 to 5.0; preferably from 3.0 to 4.5; more preferably from 3.0 to 4.0; from 3.1 to 3.9; from 3.4 to 3.8; from 3.5 to 3.7. In one embodiment, the aqueous composition of the invention has a pH equal to 3.0, 3.1, 3.2, 3.3, 3.4, 3.5, 3.6, 3.7, 3.8, 3.9, 4.0, 4.1, 4.2, 4.3, 4.4 or 4.5. The range of pH used for the composition of the invention is compatible with injection by intravenous routes and advantageously ensures a good stability of the suxamethonium composition during its shelf life. In one embodiment, the pH of the aqueous composition is adjusted between 3.5 and 3.7 during the manufacturing process of the composition, i.e. after dissolving the constituents of the composition in water. In one embodiment, the composition of the invention is sterilized by heat sterilization, preferably by steam sterilization, and in such case, the pH after sterilization is preferably ranging from 3.4 to 3.8. The pH of the composition varies during its shelf life and preferably remains within the range 3.0 to 4.5. In one embodiment, the pH of the composition on its expiration date is ranging from 3.0 to 4.5.

In one embodiment, the aqueous composition of the invention further comprises a pH adjusting agent, preferably selected from hydrochloric acid, sodium hydroxide and mixtures thereof.

In one embodiment, the aqueous composition of the invention has an osmolality ranging from 250 mOsm/kg to 350 mOsm/kg, preferably an osmolality of about 300 mOsm/kg. Preferably, this osmolality is obtained by adding a tonicity agent in the composition, such as sodium chloride. In one embodiment, the aqueous composition of the invention comprises a tonicity agent; preferably sodium chloride, preferably sodium chloride at a concentration of about 7 mg/ml. In one embodiment, when the suxamethonium composition of the invention is conditioned in a ready-to-use prefilled syringe, the osmolality is adjusted to about 300 mOsm/kg in order to provide an isotonic composition.

In one embodiment, the water used in the aqueous composition of the invention is selected from "water for injection" grade water, distilled water, purified water, milliQ water, and reverse osmosis-purified water. In one embodiment, the water used in the aqueous composition of the invention is of "water for injection" grade.

In one embodiment, the aqueous composition of the invention consists of:
- suxamethonium chloride;
- succinic acid;
- optionally a tonicity agent, such as sodium chloride;
- optionally one or more pH adjusting agent, preferably selected from hydrochloric acid, sodium hydroxide and mixtures thereof; and
- water.

In one embodiment, the aqueous composition of the invention consists of:
- suxamethonium chloride at a concentration ranging from 8 mg/ml to 12 mg/ml;
- succinic acid at a concentration ranging from 5 mM to 20 mM;
- optionally a tonicity agent, such as sodium chloride;
- optionally one or more pH adjusting agent, preferably selected from hydrochloric acid, sodium hydroxide and mixtures thereof; and
- water.

In one embodiment, the aqueous composition of the invention consists of:
- suxamethonium chloride at a concentration ranging from 8 mg/ml to 12 mg/ml;
- succinic acid at a concentration ranging from 5 mM to 20 mM;
- sodium chloride, preferably at a concentration of about 7 mg/ml;
- optionally one or more pH adjusting agent, preferably selected from hydrochloric acid, sodium hydroxide and mixtures thereof; and
- water,
and having a pH ranging from 3.0 to 4.5.

In one embodiment, the aqueous composition of the invention is sterile. Advantageously, the sterility is obtained by heat sterilization, more preferably by terminal heat sterilization. Indeed, according to ICH and EMA guidelines, terminal heat sterilization should be privileged in order to ensure a high security for patients and insurance of sterility of the final drug product. In one embodiment, the heat sterilization is performed by steam sterilization, preferably saturated steam sterilization. Preferably, the temperature for steam sterilization is ranging from 118°C to 125°C; more preferably, the temperature for steam sterilization is 121°C. Preferably, steam sterilization is performed with F0 ranging from 8 to 30; preferably from 10 to 30; more preferably from 15 to 30; from 17 to 26. In one embodiment, steam sterilization is performed with F0 of 8, 9, 10, 11, 12, 13, 14, 15, 16, 17, 18, 19, 20, 21, 22, 23, 24, 25, 26 27, 28, 29 or 30. The F0 value of a steam sterilization process is its lethality expressed in terms of the equivalent exposure time in minutes at a temperature of 121°C delivered by the process to the load in its container with reference to micro-organisms possessing a theoretical Z-value of 10 (Z-value is the change of temperature required to alter the D-Value by a factor of 10), i.e. F0 is the equivalent exposure time in minutes at 121°C of the actual exposure time at a variable temperature, calculated for an ideal microorganism with a temperature coefficient of destruction equal to 10.

Suxamethonium is known to be sensitive to temperature. Therefore, when the composition is sterilized by heat sterilization, a few part of the suxamethonium originally present in the composition is degraded during this process. It was evidenced that the percentage of degradation ranges from 3% to 5% of the original amount of suxamethonium in the composition. In order to compensate this degradation due to sterilization, an overage of suxamethonium can be introduced in the composition before sterilization, in order to reach the targeted concentration after sterilization.

In one embodiment, the suxamethonium composition of the invention remains stable overtime. In the present application, "remain stable" means that the concentration in suxamethonium in the composition remains overtime of at least 90% of the initial concentration of suxamethonium present in the composition. By "initial concentration", it is referred to the concentration of suxamethonium in the composition at its release under commercial form. Especially, when the composition is sterilized by heat sterilization, preferably by steam sterilization, the "initial concentration" of suxamethonium refers to the concentration of suxamethonium in the composition after heat sterilization. The stability can be monitored by measuring the variations of the concentrations of suxamethonium and of its products of degradation, including succinylmonocholine and choline. The concentrations can be measured by HPLC (high performance liquid chromatography). In one embodiment, the suxamethonium composition of the invention comprises less than 8% in weight to the total weight of suxamethonium chloride (%w/w_{suxa HCl}), of succinylmonocholine. In one embodiment, the suxamethonium composition of the invention comprises less than 8%w/w_{suxa HCl} of choline. When the composition of the invention is sterilized by heat sterilization, a controlled amount of the degradation products of suxamethonium is present at the initial time of the shelf life (i.e. after sterilization) of the composition. In such case, in one embodiment, the initial amount of succinylmonocholine in the composition ranges from 2% to 5% in weight to the total weight of suxamethonium chloride, preferably from 2%w/w_{suxa HCl} to 3%w/w_{suxa HCl}, and the initial amount of choline ranges from 1% to 5% in weight to the total weight of suxamethonium chloride, preferably from 1%w/w_{suxa HCl} to 2%w/w_{suxa HCl}. In one embodiment, after 24 months of storage, at a temperature of 2-8°C, the percentage of degradation of suxamethonium chloride ranges from 5% to 10% of the initial amount of suxamethonium chloride present in the composition after sterilization, preferably ranges from 5% to 8%. In one embodiment, after 24 months of storage, at a temperature of 2-8°C, the percentage of succinylcholine formed in the composition is ranging from 5% to 8% of the initial amount of succinylcholine present in the composition after sterilization; preferably ranges from 5% to 7%. In one embodiment, after 24 months of storage, at a temperature of 2-8°C, the percentage of choline formed in the composition is ranging from 4% to 8% of the initial amount of choline present in the composition after sterilization, preferably ranges from 4% to 6%.

In one embodiment, the suxamethonium composition of the invention can be stored at a temperature ranging from 2°C to 30°C, preferably from 2°C to 25°C. Preferably, the composition is stored at a temperature ranging from 2°C to 8°C. Depending on the temperature of storage, the time of storage can vary. In one embodiment, when the composition of the invention is stored at a temperature ranging from 2°C to 8°C, it remains stable for at least 1 year, preferably at least 2 years. In one embodiment, when the composition of the invention is stored at a room temperature, i.e. at a temperature of 25°C, it remains stable for at least 1 month, preferably at least 3 months. In one embodiment, the composition of the invention remains stable when stored at a temperature ranging from 2°C to 8°C during 24 months, followed by a storage at room temperature, i.e. at a temperature of about 25°C, for at least 1 month.

In one embodiment, the composition of the invention does not need an antioxidant excipient. Indeed, it was evidenced that suxamethonium is not sensitive to oxidizing conditions.

In one embodiment, the composition of the invention does not comprise a preservative agent. Indeed, the composition can be rendered sterile by heat sterilization. In the present application, "preservative agent" refers to a substance that is added to a composition in order to retard and/or prevent microbial growth. In one embodiment, the composition of the invention does not comprise methyl-4-hydroxybenzoate.

In one embodiment, the composition of the invention is disposed within a sealed container or vessel. Preferably, the sealed container or vessel has a volume selected from 5 ml, 10 ml, 20 ml and 50 ml; preferably a volume of 10 ml. In one embodiment, the sealed container or vessel is part of an injection device, preferably is a syringe or a syringe barrel.

### Suxamethonium prefilled syringe

The invention also relates to a syringe comprising a suxamethonium composition, preferably the suxamethonium aqueous pharmaceutical composition of the invention. In one embodiment, the invention relates to a suxamethonium prefilled syringe (PFS), comprising the composition of the invention.

In one embodiment, the syringe used for the suxamethonium prefilled syringe of the invention is a plastic syringe; preferably a syringe made of polypropylene (PP), cyclic olefin copolymer(COC) and/or cyclic olefin polymer (COP). A plastic syringe is particularly advantageous over a glass syringe since it is not breakable and it enables a better Luer Lock connectivity.

In one embodiment, the syringe has a total volume of selected from 5 ml, 10 ml, 20 ml and 50 ml; preferably a volume of 10 ml.

In one embodiment, the prefilled syringe of the invention has a Luer Lock male connector at the end of the syringe allowing a secured connection with any needle, transfer set, catheter or any other compatible female port for needle-free administration or reconstitution.

In one embodiment, the syringe used to manufacture the prefilled syringe of the invention is a syringe as described in EP1919537 or in EP1973592, whose contents are herein entirely incorporated by reference.

In one embodiment, the syringe used to form the prefilled syringe of the invention is a syringe as described in EP1973592. In one embodiment, the prefilled syringe is sealed at its end by a frangible obturator which is injection molded with the barrel of the syringe (see figures 2 and 3 of EP1973592). In one embodiment, the frangible obturator is covered by a protective cap (figures 5 and 6 of EP1973592). Advantageously, this protective cap both enable to protect the Luer Lock from contamination and to break the frangible obturator by its rotation. In one embodiment, a simple rotation of the protective cap breaks the frangible obturator and opens the tip of the syringe (figures 8 and 9 of EP1973592). After opening and removal of the protective cap, the Luer Lock male connector at the end of the syringe allows a secured connection with any transfer set, catheter or any other compatible female port for needle-free administration or reconstitution.

In one embodiment, the suxamethonium prefilled syringe of the invention is adapted to single dose administration.

In one embodiment, the prefilled syringe of the invention is sterile. Preferably, the sterility is obtained by heat sterilization, more preferably by terminal heat sterilization. In one embodiment, the heat sterilization is performed by steam sterilization. Preferably the temperature for steam sterilization is ranging from 118°C to 125°C; more preferably is of 121°C. Preferably, steam sterilization is performed with F0 ranging from 8 to 30; preferably from 10 to 30; more preferably from 15 to 30; from 17 to 26.

For steam sterilization to meet regulatory requirements (guaranteed sterility even in the event of heavy initial contamination), it must be carried out at a temperature of at least 121°C in so-called "wet" heat, which means that all parts of the prefilled syringe requiring sterilization must be in contact with the steam. When the prefilled syringe is packaged in a blister pack, the steam arrives from the autoclave chamber after passing through the paper seal of the package, and also comes from the vaporized contents of the syringe.

### Process of manufacturing

The invention also provides a process for manufacturing the suxamethonium composition and the suxamethonium prefilled syringe of the invention.

In one embodiment, the process for manufacturing the suxamethonium composition of the invention comprises:
a) dissolving the components of the composition in water;
b) if necessary, adjusting the pH of the solution obtained in step a) with a pH adjusting agent; and
c) filtering the solution obtained in step b) to provide the suxamethonium composition of the invention.

In one embodiment, the components of the composition are suxamethonium chloride, succinic acid and optionally sodium chloride. The suxamethonium chloride can be under the form of suxamethonium chloride anhydrous or suxamethonium chloride dihydrate. The amount of suxamethonium chloride anhydrous or dehydrate is adapted depending on the targeted amount of suxamethonium chloride in the composition.

The embodiments relative to the suxamethonium composition described above apply to the manufacturing process of the composition.

In one embodiment, the process for manufacturing the suxamethonium composition comprises a subsequent step of sterilization, preferably heat sterilization. In one embodiment, the step of heat sterilization is performed by steam sterilization, preferably the temperature ranging from 118°C to 125°C; more preferably is of 121°C; and preferably with F0 ranging from 8 to 30; preferably from 10 to 30; more preferably from 15 to 30; from 17 to 26.

In one embodiment, the invention also provides a process for manufacturing a suxamethonium prefilled syringe comprising:
i) manufacturing the suxamethonium composition of the invention according to the process mentioned above;
ii) filling a syringe with the composition obtained in step i); and
iii) plugging the filled syringe obtained in step ii) to provide the suxamethonium prefilled syringe of the invention.

The embodiments relative to the prefilled syringe described above apply to the manufacturing process of the prefilled syringe.

In one embodiment, the process for manufacturing the suxamethonium prefilled syringe comprises a subsequent step of packaging the prefilled syringe in individual blister. Preferably, the blister pack comprises a thermoformed plastic part closed by a peel-off paper seal. This paper possesses the property of being permeable to water vapor but largely impassable to microorganisms.

In one embodiment, the process for manufacturing the suxamethonium prefilled syringe comprises a subsequent step of terminal sterilization, preferably terminal heat sterilization. Preferably, the sterilization step is performed after that the prefilled syringe has been packaged in individual blister. In one embodiment, the step of heat sterilization is performed by steam sterilization, preferably the temperature ranging from 118°C to 125°C; more preferably is of 121°C; and preferably with F0 ranging from 8 to 30; preferably from 10 to 30; more preferably from 15 to 30; from 17 to 26.

### BRIEF DESCRIPTION OF THE DRAWINGS

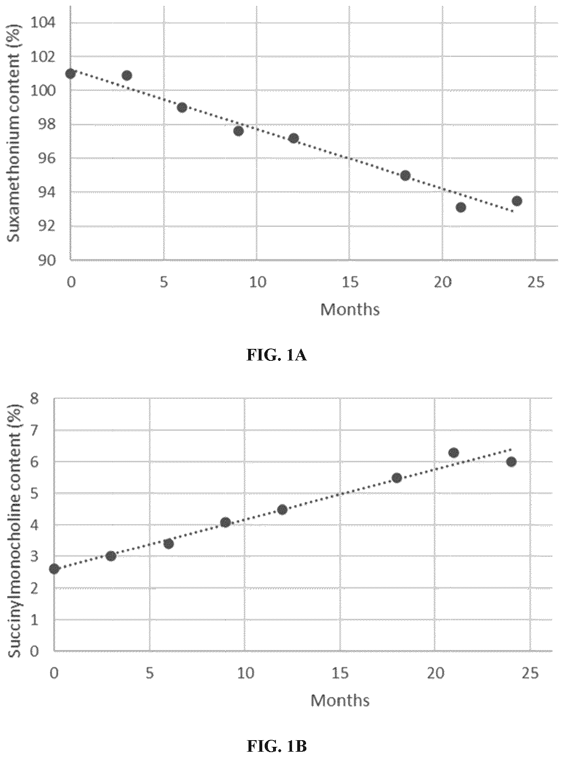

**Figures 1A** and **1B** are graphs representing the evolution overtime of the concentrations in suxamethonium (Fig. **1A**) and in succinylmonocholine (Fig. **1B**) upon storage of the composition of Example 1 at 2-8°C up to 24 months.

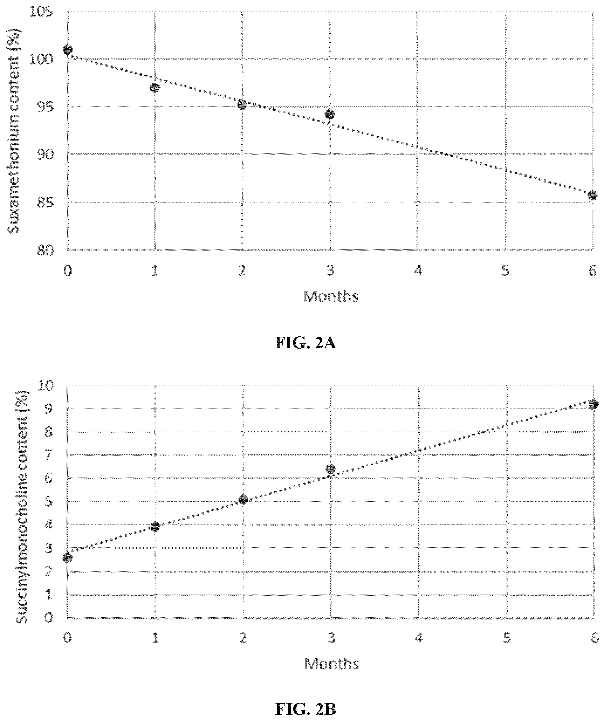

**Figures 2A** and **2B** are graphs representing the evolution overtime of the concentrations in suxamethonium (Fig. **2A**) and in succinylmonocholine (Fig. **2B**) upon storage of the composition of Example 1 at 25°C up to 6 months.

### EXAMPLES

The present invention is further illustrated by the following examples.

### Abbreviations:

- g:: grams;
- L:: liter;
- mg:: milligrams;
- ml:: milliliters;
- mM:: millimolar;
- PFS:: prefilled syringe;
- Qs:: quantum sufficiat.

### Example 1: Suxamethonium composition and prefilled syringe (PFS)

*Purpose:* Manufacturing a suxamethonium composition comprising a target concentration of 10 mg/ml of suxamethonium chloride anhydrous after sterilization. In order to compensate the degradation of suxamethonium chloride during the step of sterilization, a composition comprising 5% overage suxamethonium chloride is first prepared.

*Method:* A solution comprising the components listed in Table 1 was prepared by dissolution of suxamethonium chloride dihydrate, sodium chloride and succinic acid in water:

**Table 1. Suxamethonium composition**

| **Product** | **Composition per liter of composition** |
|---|---|
| Suxamethonium chloride dihydrate | 11.55 g |
| (Equivalent to Suxamethonium chloride anhydrous) | (10.5 g) |
| Sodium chloride | 7 g |
| Succinic acid | 720 mg |
| NaOH 1N | Qs pH 3.6 |
| HCl 1N | Qs pH 3.6 |
| Water for injection (WFI) | 1 L |

The original concentration of suxamethonium chloride anhydrous in the composition is thus 10.5 mg/ml, corresponding to an overage of 5%. The concentration of succinic acid in the composition is 6 mM.

After dissolution, the pH of the solution was adjusted to 3.6 using NaOH and/or HCl. The solution was filtered, filled in a 10 ml polypropylene syringe. After plugging the syringe, the PFS was sterilized by autoclaving (121°C, F0≥ 15). The composition of the sterilized PFS was analyzed by HPLC.

*HPLC conditions.* HPLC analyses were conducted on HPLC system Waters Alliance (with column heater and DAD 2998 detector). The chromatographic conditions are described below:

| | | |
|---|---|---|
| Column | Symmetry C18 250*4.6mm 5µm (WATERS ref WAT054275) | |
| Mobile phase composition | Buffer sodium pentanesulfonate and sodium chloride with 1% v/v sulfuric acid (filtered at 0.45µm) | |
| | Acetonitrile | |
| Composition | % Buffer | % Acetonitrile |
| | 93 | 7 |
| Needle wash | Purified water / acetonitrile 90/10 (V/V) | |
| Flow rate | 1 ml/min | |
| Column temperature | 25°C | |
| Sample temperature | 5°C | |
| Injection volume | 10 µL | |
| Detection | 214 nm | |
| Integration mode | Area | |
| Run time | 10 minutes | |
| sample | injected such as | |

*Results:* The concentration in suxamethonium chloride in the sterilized prefilled syringe was measured just after sterilization and was found to correspond to the targeted dose, i.e. 10 mg/ml in suxamethonium chloride anhydrous.

### Example 2: Stability of the suxamethonium composition

*Purpose:* Determining the stability overtime of the composition present in the sterilized PFS obtained in Example 1.

*Method:* Sterilized PFS obtained as described in Example 1 were stored at 5°C or 25°C. The concentrations in suxamethonium and its degradation products, succinylmonocholine and choline, were measured by HPLC at different time points, up to 24 months of storage.

**Storage conditions:**

| **Test** | **Temperature** | **Relative Humidity** |
|---|---|---|
| 5°C | 5 °C ± 2 °C | N.A. |
| 25°C/60%HR | 25 °C ± 2 °C | 60% |

**Stability time points:**

| | **Time (in months)** | | | | | | | | | |
|---|---|---|---|---|---|---|---|---|---|---|
| **Test** | **0** | **1** | **2** | **3** | **6** | **9** | **12** | **18** | **21** | **24** |
| 5°C | x | | | x | x | x | x | x | x | x |
| 25°C/60%HR | x | x | x | x | x | | | | | |

Time T=0 corresponds to the composition just after sterilization.

HPLC analytical conditions and equipment are identical to those of Example 1.

*Results*: The evolution of the concentration in suxamethonium, succinylmonocholine and choline upon storage at 2-8°C or 25°C is reported in Tables 2 and 3 respectively. The evolution of the concentration in suxamethonium and succinylmonocholine upon storage of the composition at 2-8°C up to 24 months is reported in Figures 1A and 1B respectively. The evolution of the concentration in suxamethonium and succinylmonocholine upon storage of the composition at 25°C up to 6 months is reported in Figures 2A and 2B respectively.

**Table 2. Stability results for storage at 5°C**

| | Shelf life specifications | T0 | T3 | T6 | T9 | T12 | T18 | T21 | T24 |
|---|---|---|---|---|---|---|---|---|---|
| pH | 3.0-4.5 | 3.6 | 3.5 | 3.5 | 3.6 | 3.5 | 3.5 | 3.5 | 3.5 |

| Suxamethonium chloride anhydrous | | | | | | | | | |
|---|---|---|---|---|---|---|---|---|---|
| mg/ml | 9.00-10.50 | 10.10 | 10.09 | 9.90 | 9.76 | 9.72 | 9.50 | 9.31 | 9.35 |
| %/Theory | 90.0 - 105.0 | 101.0 | 100.9 | 99.0 | 97.6 | 97.2 | 95.0 | 93.1 | 93.5 |
| Succinylmonocholin e HCl (%w/w_{suxa HCl}) | ≤ 8.0 | 2.6 | 3.0 | 3.4 | 4.1 | 4.5 | 5.5 | 6.3 | 6.0 |
| Choline HCl (%w/w_{suxa HCl}) | ≤ 8.0 | 1.7 | 2.1 | 2.4 | 3.1 | 3.3 | 4.3 | 4.9 | 4.5 |

**Table 3. Stability results for storage at 25°C**

| | Shelf life specifications | T0 | T1 | T2 | T3 | T6 |
|---|---|---|---|---|---|---|
| pH | 3.0-4.5 | 3.6 | 3.5 | 3.5 | 3.4 | 3.4 |

| Suxamethonium chloride anhydrous | | | | | | |
|---|---|---|---|---|---|---|
| mg/ml | 9.00-10.50 | 10.10 | 9.70 | 9.52 | 9.42 | 8.57 |
| %/Theory | 90.0 - 105.0 | 101.0 | 97.0 | 95.2 | 94.2 | 85.7 |
| Succinylmonocholine HCl (%w/w_{suxa HCl}) | ≤ 8.0 | 2.6 | 3.9 | 5.1 | 6.4 | 9.2 |
| Choline HCl (%w/w_{suxa HCl}) | ≤ 8.0 | 1.7 | 3.1 | 4.3 | 5.4 | 8.6 |

*Discussion*: The stability data at T24 months at 2-8°C indicates:
- suxamethonium content: 93.5% of the initial content in suxamethonium; and
- succinylmonocholine content: 4.9% w/w.

This is well within the maximum specifications of 90% suxamethonium content and 8% succinylmonocholine. The tested composition thus presents an optimized stability and a targeted shelf life of minimum 24 months at 2-8°C with this formulation.

The stability data at T3 months at 25°C indicates:
- suxamethonium content: 94.2% of the initial content in suxamethonium; and
- succinylmonocholine content: 6.4% w/w.

This is also well within the maximum specifications of suxamethonium and succinylmonocholine. The tested composition thus presents an optimized stability and a targeted shelf life of minimum 3 months at 25°C with this formulation.

### Example 3: Degradation during sterilization and corresponding needed overage

*Purpose:* Suxamethonium is known to be a quite unstable molecule and to degrade when exposed to heat. However, terminal heat sterilization of drugs should be privileged in order to ensure a high security for patients and insurance of sterility of the final drug product. Therefore, the degradation of suxamethonium under heat sterilization was studied in order to determine the overage of the composition required before sterilization in order to compensate degradation.

*Method*: Several batches of target composition (i.e. comprising 10 mg/ml of suxamethonium chloride anhydrous) have been manufactured and were subjected to steam sterilization at 121.3°C on a pilot autoclave (Autoclave Lequeux) or on an industrial equipment (Autoclave SBM 58EQ002, model SDR-10.09.50).

The loss of suxamethonium as well as the formation of the degradation products succinylmonocholine and choline were measured by HPLC at T0, i.e. just after the sterilization step. HPLC analytical conditions and equipment are identical to those of Example 1.

*Results:* The results are presented in Table 4:

**Table 4. Effects of steam sterilization**

| | | **Degradation products** | |
|---|---|---|---|
| | **Loss in suxamethonium (%)** | **% w/w succinyl-monocholine** | **% w/w choline** |
| **Pilot autoclave: F0 18** | 3.2 | 1.2 | Not analyzed |
| **Pilot autoclave: F0 25** | 3.3 | 1.9 | Not analyzed |
| **Pilot autoclave: F0 26** | 3.7 | 2.3 | Not analyzed |
| **Pilot autoclave: F0 25** | 3.6 | 1.8 | 1.3 |
| **Industrial autoclave: mean cycle** | 3.2 | 1.8 | 1.3 |

Mean degradation on pilot scale is approximately 3.4%. The degradation with the industrial cycle is of the same order of magnitude. Pilot autoclave cycles were conducted at the upper limit to ensure most stringent conditions. The temperature increase and decrease on the industrial equipment is slightly slower due to thermal inertia. The conducted cycle was conducted at the standard (mean) conditions. If the cycle is conducted at the upper limit, degradation will be slightly higher.

*Discussion:* The degradation products of suxamethonium are well identified and the amount of active substance degradation decrease is identical equivalent to the amount of degradation products appearing upon HPLC and ionic chromatography analysis (sum of succinylmonocholine, choline and succinic acid provides the correct mass balance). These results are in accordance with scientific literature that describes the degradation of suxamethonium upon exposure to heat. It has to be mentioned that the degradation products of suxamethonium are its natural metabolites (upon degradation in vivo). Moreover, a safety assessment has proved (based on bibliographic data) that the degradation products at the maximum concentrations achievable do not present a significant pharmacological or toxicological effect. They are safe and without danger to be injected at these concentrations.

In order to ensure the right suxamethonium dosage upon batch release, the suxamethonium overage in the composition can be fixed at 5% during manufacture. This overage takes into account the degradation during the step of sterilization, but also the overall degradation that occurs during the cumulated holding time during manufacturing which is performed at a temperature of about 25°C. With this overage, the degradation that can occurs during the 24 months shelf life of the composition stored at 2-8°C, during transportation and during a possible final storage before use for a 4 weeks period at room temperature, in the worst conditions for each situation, will not exceed the minimum specifications of 90% suxamethonium content.

### Example 4: Effect of the amount of succinic acid on the stability of the composition

*Purpose:* Determining the effect of the amount of succinic acid present in the composition on its stability overtime.

### Methods and results:

### 1) Need of a pH buffer:

Two formulations at 10 mg/ml suxamethonium chloride, with and without 20 mM succinic acid buffer, were prepared and adjusted to pH 4.5. After steam sterilization, samples were stored at 25°C for two weeks. The unbuffered solution exhibits a significant pH drop in less than 2 weeks, contrary to the succinic acid buffered composition (results in Table 5). This will thus lead to out of specification pH before reaching the shelf life. Thus, a pH buffer is required to maintain the target pH during the whole shelf life.

**Table 5. pH of unbuffered composition compared to succinic acid buffered composition, after 2 weeks storage at 25°C**

| | Without pH buffer | With 20 mM succinic acid buffer |
|---|---|---|
| pH t0 | 4.5 | 4.5 |
| pH t14 days | 4.02 | 4.43 |

### 2) Comparison of compositions comprising 20 mM or 50 mM of succinic acid at three different pH values:

Formulations at 10 mg/ml suxamethonium chloride, comprising 20 mM or 50 mM of succinic acid were prepared and adjusted at pH 3.0, 3.5 or 4.5. After steam sterilization, samples were stored at 25°C for one week and the suxamethonium content was measured by HPLC (results in Table 6).

**Table 6. Suxamethonium content (%), depending on pH and succinic acid concentration, after 1-week storage at 25°C**

| | pH 3.0 | pH 3.5 | pH 4.5 |
|---|---|---|---|
| Without succinic acid | 99.1 | 99.3 | 99.0 |
| 20mM succinic acid | 98.9 | 98.5 | 97.7 |
| 50mM succinic acid | 97.8 | 96.8 | 90.5 |

After one-week storage at 25°C, stability of suxamethonium is similar at pH 3.0 and 3.5, while stability decreases at pH 4.5. In these conditions, chemical stability is similar with and without 20mM succinic acid. However, as evidenced above, succinic acid is required to maintain pH stability beyond one week of storage at 25°C. 50 mM succinic acid leads to faster degradation rate. Thus, the amount of succinic acid was adjusted to a maximum at 20 mM. The succinic acid content can be minimized and should enable a proper control of pH value all over the product shelf life.

### 3) Comparison of compositions comprising 4 mM or 6 mM of succinic acid:

Formulations at 10 mg/ml suxamethonium chloride, comprising 4 mM or 6 mM of succinic acid were prepared and adjusted at pH 3.6. After steam sterilization, samples were stored at 25°C up to 4 months and the pH was monitored (Table 7).

**Table 7. pH of composition comprising 4 mM or 6 mM of succinic acid upon storage at 25°C**

| | 4 mM succinic acid buffer | 6 mM succinic acid buffer |
|---|---|---|
| pH adjustment | 3.6 | 3.6 |
| T0 after steam sterilization | 3.4 | 3.5 |
| T1 month | 3.3 | 3.5 |
| T2 months | 3.2 | 3.4 |
| T4 months | 3.2 | 3.4 |

Above data demonstrate that the pH decrease with 4 mM succinic acid is faster than with 6 mM succinic acid. Consequently, a concentration of 4 mM of succinic acid is not sufficient to control the pH decrease (especially if the initial pH is at the lower end of the specification).

*Discussion*: The degradation of suxamethonium chloride proceeds through a hydrolysis of the ester bonds. The hydrolysis yields succinylmonocholine, choline and succinic acid. The formation of succinic acid upon hydrolysis leads to pH decrease. The Applicant found that buffering the composition enables to avoid an out of specification pH at shelf life and an acceleration of the degradation upon pH drop.

The buffer chosen for the composition of the invention is succinic acid. It was selected at least for the following reasons:
- succinic acid is an approved and widely accepted pharmaceutical excipient;
- succinic acid has a high buffer capacity around the targeted pH value of pH 3.6;
- succinic acid is also one of the degradation products and metabolites of suxamethonium chloride.

The above results show that the concentration of succinic acid has to be carefully selected: the composition should comprise enough but not too much succinic acid in order to achieve expected stability during the shelf life. A range of 5 mM to 20 mM was found to provide expected effects. Higher succinic acid content increases the degradation kinetic of suxamethonium. Lower succinic acid content negatively impacts the buffering of the composition and the degradation of suxamethonium.

## Claims

1. An aqueous pharmaceutical composition for parenteral administration, comprising:
- suxamethonium chloride at a concentration ranging from 8 mg/ml to 12 mg/ml; and
- succinic acid at a concentration ranging from 5 mM to 20 mM;
and having a pH ranging from 3.0 to 4.5.

2. The aqueous pharmaceutical composition according to claim 1, wherein the concentration of suxamethonium chloride in the composition corresponds to 10 mg/ml of suxamethonium chloride anhydrous.

3. The aqueous pharmaceutical composition according to claim 1 or claim 2, wherein succinic acid is present at a concentration of 6 mM.

4. The aqueous pharmaceutical composition according to any one of claims 1 to 3, wherein the pH is ranging from 3.4 to 3.8.

5. The aqueous pharmaceutical composition according to any one of claims 1 to 4, wherein the composition has an osmolality ranging from 250 mOsm/kg to 350 mOsm/kg, preferably an osmolality of about 300 mOsm/kg.

6. The aqueous pharmaceutical composition according to any one of claims 1 to 5, further comprising sodium chloride, preferably at a concentration of about 7 mg/ml.

7. The aqueous pharmaceutical composition according to any one of claims 1 to 6, further comprising a pH adjusting agent, preferably selected from hydrochloric acid, sodium hydroxide and mixtures thereof.

8. The aqueous pharmaceutical composition according to any one of claims 1 to 7, wherein the water used to form the aqueous composition is water for injection grade.

9. The aqueous pharmaceutical composition according to any one of claims 1 to 8, being sterile, the sterility being obtained preferably by heat sterilization.

10. A suxamethonium prefilled syringe comprising an aqueous pharmaceutical composition according to any one of claims 1 to 9.

11. The suxamethonium prefilled syringe according to claim 10, wherein the syringe is a plastic syringe; preferably a syringe made of polypropylene, cyclic olefin copolymer and/or cyclic olefin polymer.

12. The suxamethonium prefilled syringe according to claim 10 or claim 11, wherein the syringe has a total volume of selected from 5 ml, 10 ml, 20 ml and 50 ml.

13. The suxamethonium prefilled syringe according to any one of claims 10 to 12, being sterilized by terminal heat sterilization.

14. A process for sterilizing a suxamethonium prefilled syringe, comprising:
- providing a suxamethonium prefilled syringe according to any one of claims 10 to 13;
- subjecting the suxamethonium prefilled syringe to steam sterilization at a temperature ranging from 118°C to 125°C, with F0 ranging from 8 to 30.
